(19) Europäisches Patentamt
European Patent Office
Office européen des brevets

(11) EP 1 371 630 B1

(12) EUROPEAN PATENT SPECIFICATION

(45) Date of publication and mention of the grant of the patent:
**01.10.2008 Bulletin 2008/40**

(51) Int Cl.:
*C07C 229/24* (2006.01) *C07C 227/24* (2006.01)

(21) Application number: **03013165.0**

(22) Date of filing: **11.06.2003**

# (54) Method for preparing a stable aqueous aminopolycarboxylic acid solution composition

Methode zur Herstellung einer stabilen wässrigen Aminopolycarbonsäurelösung zusammensetzung

Méthode de préparation d'une composition de solution aqueuse stable d'acide aminopolycarboxylique

(84) Designated Contracting States:
**BE DE**

(30) Priority: **11.06.2002 JP 2002170205**

(43) Date of publication of application:
**17.12.2003 Bulletin 2003/51**

(73) Proprietor: **NIPPON SHOKUBAI CO., LTD.**
**Osaka-shi, Osaka 541-0043 (JP)**

(72) Inventors:
- **Sumida, Yasutaka**
**Neyagawa-shi,**
**Osaka 572-0051 (JP)**
- **Kitajima, Mitsuhiro**
**Suita-shi,**
**Osaka 564-0035 (JP)**

(74) Representative: **Brehm, Hans-Peter et al**
**Hansmann & Vogeser**
**Patent- und Rechtsanwälte**
**Postfach 1164**
**82301 Starnberg (DE)**

(56) References cited:
**EP-A- 0 708 078**

- **DATABASE HCAPLUS ACS; 17 April 1998 (1998-04-17), XP002279575 retrieved from STN Database accession no. 128:296179/DN & JP 10 087580 A (NITTO CHEMICAL IND.) 7 April 1998 (1998-04-07)**
- **DATABASE HCAPLUS ACS; 17 April 1998 (1998-04-17), XP002279576 retrieved from STN Database accession no. 128:283911/DN & JP 10 087582 A (NIPPON CHEMICAL IND.) 7 April 1998 (1998-04-07)**
- **DATABASE WPI Section Ch, Week 199542, Derwent Publications Ltd., London, GB; Class D25, AN 1995-325515 -& JP 07 224014 A (NITTO CHEM. IND. CO.) 22 August 1995**
- **DATABASE WPI Week 199845, Derwent Publications Ltd., London, GB; Class D25, AN 1998-525608 -& JP 10 231469 A (NITTO CHEM. IND. CO.) 02 September 1998**
- **DATABASE WPI Week 199727, Derwent Publications Ltd., London, GB; Class D25, AN 1997-294926 -& JP 09 110812 A (MITSUBISHI CHEM. CORP.) 28 April 1997**
- **DATABASE WPI Week 200275, Derwent Publications Ltd., London, GB; Class B05, AN 2002-693597 -& JP 2002 088034 A (NIPPON SHOKUBAI CO.) 27 March 2002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 371 630 B1

## Description

The present invention relates to a method for preparing an aqueous aminopolycarboxylate solution composition containing one or more aminopolycarboxylate compound(s) represented by the following general formula (1):

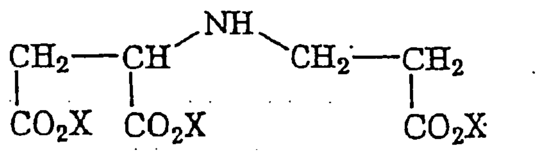

(1)

wherein:

X may be the same or different and represents a hydrogen atom, an alkali metal atom, or an ammonium group.

BACKGROUND OF THE ART

Salts of aminopolycarboxylic acids such as N-2-carboxyethyl-aspartic acid (aminopolycarboxylates) have high chelating activity in order to sequester metal ions and may find application as chelating agents for use in the removal of toxic metals, concentrating and recovery of valuable metals, and fractional purification of metals.

However, when handled in solid state such as powder and granules, aminopolycarboxylates absorb atmospheric moisture to form viscous masses, the handling and further processing thereof provides difficulties.

Aqueous aminopolycarboxylate solutions of low purity tend to precipitate and to form deposits; accordingly, the aminocarboxylate content in the aqueous solution must be kept sufficiently low.

However, if aminopolycarboxylates could be handled in a stable manner, these compounds should become applicable to industrial uses such as detergent builders, soap additives, textile dyeing agents, plating chemicals, auxiliary bleaches, and photographic chemicals (photographic chelating agents) for the formulation of photographic processing compositions. Therefore, there is a demand for providing aminopolycarboxylates in a stabilized form and manner.

Japanese Kokai Publication Hei-08-169866 discloses an aqueous solution of an iminocarboxylate salt controlled in such a manner that:

- a molar ratio of the D-form : L-form of an aspartic acid skeleton of said iminocarboxylate salt is in the range of from 1 : 0 to 0.7 : 0.3 or in the range of from 0 : 1 to 0.3 : 0.7;
- the content of said iminocarboxylate salt amounts of from 40 to 70 weight % and
- the solution comprises a pH value of from 7 to 12.

Both, Japanese Patent Publication No. 2644977 and the corresponding document EP 0 708 078 A1 disclose an aqueous solution of iminocarboxylate salt(s) having a molar ratio of the D-form : L-form of the aspartic acid skeleton of said iminocarboxylate salt in the range of from 1 : 0 to 0.7 : 0.3 or in the range of from 0 : 1 to 0.3 : 0.7, wherein the content of said iminocarboxylate salt amounts of from 40 to 70 weight % and the pH value of the aqueous composition is kept in the range of from 7 to 12. When these three essential requirements are fulfilled, the iminocarboxylic acid salt can be handled as a stable and homogeneous aqueous solution for a long time without suffering either precipitation of crystals or solidification of the aqueous solution. Moreover, the aqueous iminocarboxylic acid salt solution can be easily handled. Selected iminocarboxylates of this type may be obtained by reacting an aspartate with a maleate under alkaline conditions in an aqueous medium.

However, said iminocarboxylates comprise a chemical structure according to the following general formula (III)

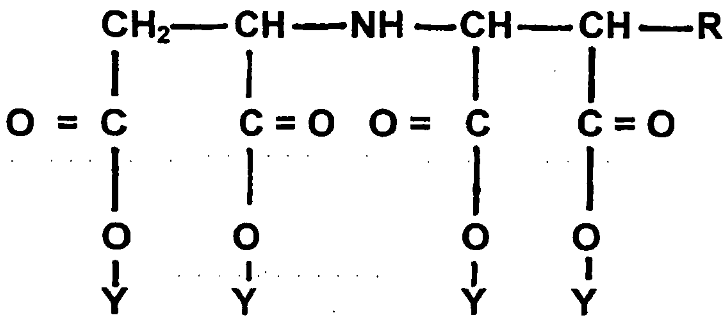

(III)

wherein:

R represents a hydrogen atom or a hydroxyl group; and
Y represents a sodium atom, a potassium atom or an ammonium group.

Accordingly, these iminocarboxylates comprise a chemical structure having two or three asymmetric carbon atoms; contrarily, the aminocarboxylates according to the present, above-stated general formula (I) comprise only one asymmetric carbon atom.

Japanese Kokai Publication Hei-10-87580 discloses a monoaminocarboxylate having a chemical structure according to the following general formula (IV)

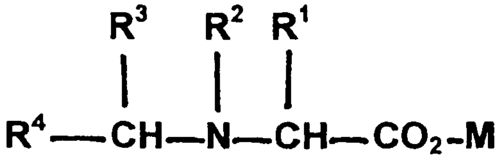

(IV)

wherein:

each of R1, R2, and R3 may represent hydrogen or a selected hydrocarbon;
R4 represents H, OH, $CO_2H$ or $SO_3M$, and
M represents hydrogen or an alkali metal.

A preferred compound is 3Na-S-aspartic acid-N-monoacetate.

The underlying carboxylic acid, for example aspartic acid -N-mono acetic acid, is obtained by performing an addition reaction of a raw amino acid with prussic acid and formaldehyde, and hydrating the thus obtained reaction product under alkaline conditions. The product is controlled to have a total content of nitrile compounds and amide compounds of 2 % or less based on the monoaminocarboxylate.

Japanese Kokai Publication Hei-10-87582 discloses similar monoaminocarboxylic acids and salts thereof, and obtained in a similar manner. The product is controlled to have - based on the monoaminocarboxylate - a total content of cyanide ion, hydrogen cyanide and salts thereof of not more than 100 ppm, a total content of ammonium ion, ammonia and salts thereof of not more than 1 %, and a content of formaldehyde of not more than 1 %.

The manufacturing process of those monoaminocarboxylates comprises the addition of prussic acid, respectively, cyanic acid and formaldehyde to the starting amino acid and subsequent hydrolysis of the obtained addition compound under alkaline conditions. The thus obtained monoaminocarboxylate composition comprises a pH value of about 13.5 to 14 and is considered to have a non sufficient stability.

Nonexamined Japanese Patent Publication No. 07-224 014 A is related to a method of preparing L-aspartic acid-N-monopropionic acid, wherein acrylonitrile is added to L-aspartic acid under alkaline conditions, and the thus obtained reaction product is treated with alkali metal hydroxide. The hereby obtained product is hydrolyzed by adding sulfuric acid.

Nonexamined Japanese Patent Publication No. 10-231469 A discloses a chelating agent which may comprise - for example - a mixture of aspartic acid and aspartic-N-2 acetic acid, or mixture of aspartic acid and aspartic acid N-1

propionic acid, and which is obtained by compounding these components. This chelating agent may form an aqueous solution.

Nonexamined Japanese Patent Publication No. 09-110812 A discloses a method of preparing a biodegradable aminopolycarboxylic acid; for example aspartic acid may be reacted with acrylic acid in an aqueous medium in the presence of an alkaline agent. A preferred reaction temperature is 50 to 100°C.

According to example 1, 14,5 g (0.35 M) sodium hydroxide were dissolved in 100 ml of water; then, 13.31 g (0.1 M) L-asparatic acid and 10.81 g (0,15 M) acrylic acid were added. The reaction mixtures were maintained for 8 hours at reflux temperature. Thereafter 35,4 g of concentrated hydrochloric acid were added, and crystallization was performed at 4 degrees C. Finally, 15,35 g N-2-carboxy ethyl-L-asparatic acid were obtained.

Nonexamined Japanese Patent Publication. No. 2002-088034 A is related to a process for preparing a N-carboxy alkyl-amino acid by reacting an amino acid, such as glutamic acid or aspartic acid, most preferred L-aspartic acid with (meth)acrylic acid in an aqueous medium in the presence of ammonia. A preferred reaction temperature is 60 to 150 °C. At least a part of the ammonia may be provided by $-COONH_4$ groups of the amino acid According to an example 49.0 g water, 60.8 g of a 28-% by weight aqueous ammonia solution, and 67.2 g L-asparatic acid were introduced into a 200 ml reactor equipped with agitator, condensator, and thermometer. Furthermore, 45.0 g of a 80-% by weight acrylic-acid water solution were added. The reaction mixture was maintained for 2 hours under reflux conditions.

## SUMMARY OF THE PRESENT INVENTION

In view of the above presented state of the art, the present invention has for its object to provide a stabilized aqueous aminopolycarboxylate solution composition stabilized in so far that the aminopolycarboxylate is hindered to precipitate and to form deposits.

The inventors as denominated to the present patent encountered several problems when trying to provide a stabilized aqueous solution of aminopolycarboxylate(s):

(1) Although it appears economical to handle the aminopolycarboxylate with high concentration and at a low temperature, the aminopolycarboxylate crystallizes under such high-concentration, low-temperature conditions. The degree of precipitation increases with the lapse of time and, in some cases, the aqueous solution as such may completely solidified.

(2) It appear that a higher content of impurities increases the extent of said precipitation.

(3) Even, in absence of crystallization when the viscosity of the aqueous solution exceeds 10 Pa·s, then the handling of the product becomes extremely difficult and the product could not easily be applied to many end-uses.

(4) When an attempt is made to heat the aqueous aminopolycarboxylate solution in order to increase the concentration of dissolved aminocarboxylate to a desired level, then the aqueous aminopolycarboxylate solution becomes stained and coloured which detracts the commercial value; further the aminopolycarboxylate is precipitated.

(5) It appears, that the stability of the aqueous aminopolycarboxylate solution could be improved when the molar ratio of the D-form : L-form of an aspartic acid skeleton of the aminocarboxylate is selected unequal to 1 : 1 which is the molar ratio of the racemix mixture.

(6) Finally, the inventors found that if a combination of features, including - the concentration of solids in the aqueous aminocarboxylate solution;

- a specified pH value of the aqueous solution;
- a specified molar ratio of D-form : L-form of the as prepared aminopolycarboxylate; and
- a specified content of polymer and other impurities produced during the production step of the aminocarboxylate (s),

is thoroughly and mutually adjusted, then the viscous precipitate will hardly form, and the aminopolycarboxylate is protected against precipitating over a prolonged period of time and can be provided in the form of an aminopolycarboxylate aqueous solution which is stable and homogeneous substantially without separation of crystals or solidification of the aqueous solution.

The present invention has been developed on the basis of these results.

Therefore, the present invention provides
a method for preparing an aqueous aminopolycarboxylate solution composition containing

one or more aminopolycarboxylate compound(s) represented by the following general formula (1):

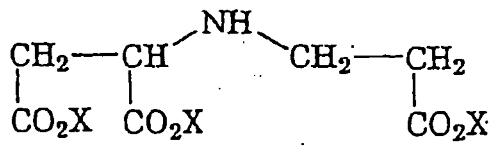

(1)

wherein:

X may be the same or different and represents a hydrogen atom, an alkali metal atom, or an ammonium group;

wherein said method comprises the steps of:

- reacting aspartic acid and/or a salt thereof with an acrylic acid compound in an aqueous medium under conditions wherein:
  - - the acrylic acid compound is added to the reaction system, and during said adding the temperature of the reaction system is kept at 50 °C or higher; and
  - - the acrylic acid compound is added to the reaction system in such a manner as to adjust the concentration of acrylic acid compound within the reaction system to 10 mass % or less;
  - - a content of an acrylic polymer matter formed as impurity from the acrylic acid compound is maintained not higher than 1 mass % based on the whole aqueous solution;
  - - the pH of reaction is adjusted to a value not higher than 11 during the reaction or after completing the reaction; and
- providing in the thus prepared aqueous aminopolycarboxylate solution composition a molar ratio of the D-form : L-form of an aspartic acid skeleton of the aminopolycarboxylate in the range of from 1 : 0 to 0.7 : 0.3 or in the range of from 0.3 : 0.7 to 0 : 1; and
  wherein the aqueous aminopolycarboxylate solution composition comprises a solid concentration of from 25 to 60 mass %.

Beneficial embodiments of this method are subject of the dependent Claims.

DISCLOSURE OF THE PRESENT INVENTION

The present invention is now described in more detail.

The aqueous aminopolycarboxylate solution composition according to the present invention contains an aminopolycarboxylate represented by the above-mentioned general formula (1), and is suited to provide such an aminopolycarboxylate in the form of a stable and homogeneous aqueous solution. In the present invention, the aminopolycarboxylate of the general formula (1) is N-2-carboxyethyl-aspartic acid and/or its salt, further, one or two or more of such compounds may be used.

Referring to the above-mentioned general formula (1), X may be the same or different and represents a hydrogen atom, an alkali metal atom, or an ammonium group. Preferably, the alkali metal atom is lithium, sodium or potassium; even more preferred is sodium. As used here, the term "aqueous solution composition" defines a composition essentially forming an aqueous solution of the aminopolycarboxylate and optionally containing other additional ingredient(s).

As used here, the term "the aspartic acid skeleton of the aminopolycarboxylate represented by the above-mentioned general formula (1)" means a structure represented by the following general formula (2) within the compound of general formula (1).

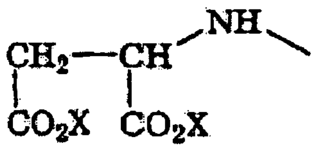

(2)

(in the formula, X may be the same or different and represents a hydrogen atom, an alkali metal atom, or an ammonium group.)

As used herein, either the L-Form or the D-Form of the aspartic acid skeleton means the compound having the asymmetric carbon in the structure represented by the general formula (2) either in the S-configuration or in the R-configuration; thus the S-configured compound comprises the L-form and the R-configured compound comprises the D-form.

Using the fore-mentioned definitions, the aqueous aminopolycarboxylate solution composition according to the present invention comprises an aminopolycarboxylate having a molar ratio of the D-form : L-form of an aspartic acid skeleton of the aminopolycarboxylate in the range of from 1 : 0 to 0.7 : 0.3 or in the range of from 0.3:0.7 to 0 : 1; preferably, this molar ratio is in the range of from 1 : 0 to 0.8 : 0.2 or in the range of from 0 : 1 to 0.2 : 0.8. If the molar ratio of the D-form to the L-form or vice versa of the aspartic acid skeleton of the aminopolycarboxylate deviates from this range, crystals separate and precipitate within a short time period from said aqueous aminopolycarboxylate solution composition in order to provide a non-homogenous slurry.

As stated above, the aqueous aminopolycarboxylate solution composition according the present invention comprises a solid concentration of from 25 to 60 mass %.

If the solid concentration of the aqueous aminopolycarboxylate solution is less than 25 mass %, then the aqueous solution may be handled as a stable aqueous solution, but due to the rather high dilution, the equipment for storage and transportation becomes large and economically disadvantageous. If the solid concentration exceeds 60 mass %, crystals will separate out from the aqueous solution composition making the handling thereof difficult. Within the above-mentioned solid concentration range, even sodium aminopolycarboxylate may be handled as a stable aqueous solution composition. The more preferred solid concentration range is not less than 35 mass % and not more than 55 mass %; and the still more preferred solid concentration is not less than 40 mass % and not more than 50 mass %. The solid referring to the solid concentration includes not only the aminopolycarboxylate but also impurities and salts thereof which will be described hereinafter.

As stated above, the pH values of the aqueous aminopolycarboxylate solution composition is adjusted to a value not higher than 11. If the pH value exceeds pH 11, staining of the aqueous solution will occur, and progressive decomposition of the aminopolycarboxylate will take place during storage which causes a product degradation. Preferably, the pH value of the aqueous solution is not lower than 4. If the pH is lower than 4, then decomposition and precipitation of the aminopolycarboxylate out from the aqueous solution may occur, making it difficult to handle the solution as a homogeneous aqueous solution. Preferably, the pH is not higher than 10.5 and not lower than 4.5. Even more preferred, the pH is not higher than 10 and not lower than 6.

When measuring the pH value, the preferred measuring temperature is 25°C and measurement may be effected with a pH meter using glass composite electrodes. The suitable glass composite electrode is 6366-10D (product name, product of HORIBA, Ltd.); the preferred pH measuring device is pH Meter F-22 (product name, product of HORIBA, Ltd.) .

The purity of the aminopolycarboxylate is expressed by the following equation:

$$\text{Purity (mass \%)} = \text{concentration of aminopolycarboxylate/solid concentration)} \times 100$$

The aqueous aminopolycarboxylate solution composition according to the present invention may not only contain aminopolycarboxylates of high purity, but may also contain aminopolycarboxylate of low purity which according to the hitherto practice is crystallized out from the aqueous solution.

The effect of stabilizing the aqueous aminopolycarboxylate solution composition according to the present invention is remarkable when the purity of the aminopolycarboxylate is 95 mass % or less. Suitable, the lower limit of the purity of the aminopolycarboxylate is not less than 60 mass %. Thus, the present invention enables to prepare a stable and homogeneous aqueous aminopolycarboxylate solution composition even when the purity of the aminopolycarboxylate is 90 mass % or less; this effect is even more remarkable and outstanding when this purity is 80 mass % or less.

The impurities referred here include unreacted aspartic acid, acrylic polymer and fumaric acid. Among these,

fumaric acid and aspartic acid show a low solubility in water and tend to precipitation. In accordance with the present invention, even when the aqueous aminopolycarboxylate solution composition contains those easily-precipitating impurities, then the overall composition may be stabilized in the form of an aqueous solution free from precipitation.

The method of preparing the aqueous aminopolycarboxylate solution according to the present invention comprises the step of reacting aspartic acid and/or a salt thereof with an acrylic acid compound in an aqueous medium. Along with this reaction an acrylic polymer may be formed from this acrylic acid compound. A content of an acrylic polymer formed as impurity from the acrylic acid compound and contained in the aqueous aminopolycarboxylate solution composition according to the invention is not higher than 1 mass % based on the whole aqueous solution. If said polymer content exceeds 1 mass %, a viscous precipitate is generated during handling the aqueous solution composition. The even more preferred polymer content is not higher than 0.5 mass %.

Preferably, the here concerned polymer content is determined by means of gel permeation chromatography (GPC). For example the following means may be used:

- as the column, SHODEX Asahipak GF-7M HQ (product name, product of Showa Denko K. K.); and
- as the carrier, a carrier solution prepared by dissolving 34.5 g of disodium hydrogen phosphate and 46.2 g of sodium dihydrogen phosphate into 5 L of ultra pure water and filtrating the solution with a 0.45 μm membrane filter (product of Advantec Toyo Kaisha, Ltd.).

Preferably, the acrylic acid compound is acrylic acid, an acrylic ester, or sodium acrylate. Both, the aspartic acid and/or a salt thereof and said acrylic acid compound may be used in the form of one or two or more different species. The ratio of aspartic acid and/or a salt thereof and acrylic acid compound in the starting material is not particularly restricted. The aqueous medium is water or a mixture of water and a solvent soluble in water, and water; further, a mixed solvent made of water with methanol, ethanol, isopropyl alcohol, acetone or acetonitrile may be used; however the preferred aqueous medium is mere water.

If the starting material comprises the D-form of aspartic acid and/or its salt, the configuration thereof will be retained as R-configuration at the asymmetric carbon atom of the aspartic acid skeleton in the structure of the resulting aminpolycarboxylate. Thus, the obtained product contains the aminopolycarboxylate in the form of the D-isomer of the aspartic acid skeleton. The method of preparing such an aminopolycarboxylate comprises the step of adding the acrylic acid compound to a reaction system containing the aspartic acid in a form wherein the functional carboxylic groups thereof have been partially or totally neutralized to give an aqueous solution containing an aminopolyaspartic acid. Preferably, said degree of neutralization of the functional carboxylic groups of the aspartic acidis adjusted to 60 to 100 %. Furthermore and preferably, the molar ratio of addition of the acrylic acid compound to the aspartic acid is set to 0.8 to 1.2. Moreover, when the acrylic acid compound is added, the reaction system is controlled to a temperature not below 50°C. Furthermore, the acrylic acid compound is added to the reaction system in such a manner as to adjust the concentration of the acrylic acid compound within the reaction system to not more than 10 mass %. Furthermore, the pH of the reaction mixture is adjusted to a value at 11 or below, during the reaction or after completing the reaction. Thus, a stable aqueous aminopolycarboxylate solution free from precipitation can be obtained.

In the above-stated preparation method, it is necessary to add the acrylic acid compound to the reaction system dropwise instead of adding it in a block-wise manner. Preferably, the duration of the dropwise addition of the acrylic acid compound is set to a period not less than 30 minutes, even more preferred to a period lasting 1 hour to 3 hours. Furthermore, said dropwise addition is effected in such a manner assuring a concentration of the acrylic acid compound in the reaction mixture not higher than 10 mass %. By such dropwise addition, the formation of the polymer during production of the aminopolycarboxylate can be inhibited, and the stability of the aqueous aminopolycarboxylate solution composition can be improved.

The adjustment of the molar ratio of the D-form : L-form or vice versa of the aspartic acid skeleton of the above aminopolycarboxylate within the provided range may be effected by using as starting material an aspartic acid and/or salt thereof having a molar ratio of D-form to L-form within the specified range. According to an alternative method, both, the D-form aminopolycarboxylate and the L-form of aminopolycarboxylate may be synthesized independently and blended thereafter in the specified ratio.

The pH value of the aqueous aminopolycarboxylate solution may be adjusted by using a combination of starting materials providing the aqueous solution having the specified pH range. Alternatively, a basic compound may be added before and/or after the reaction to adjust the pH value. Suited basic compounds include alkali metal hydroxides or carbonates and ammonium group-containing compounds those basic compounds may be used in the form of one or two or more different species. The preferred basic compound is sodium hydroxide.

In order to prevent a precipitation of crystals during the method of preparation, the starting materials may be used in such a concentration that the finally obtained product, that is the aqueous aminopolycarboxylate solution composition, contains the aminocarboxylate in a concentration lower than 30 mass %. In this case, it is necessary to concentrate the aqueous aminopolycarboxylate solution by heating in order to obtain a solid concentration of 25 to 60 mass

%, and a pH value not higher than 11. Preferably, this concentrating by heating is effected by treating an aqueous solution of pH 4 to 11 under reduced pressure at a temperature of 30 to 90°C. At any temperature exceeding 90°C, the aminopolycarboxylate is partially decomposed, and a stained product is obtained having reduced commercial value. Concentrating at a temperature lower than 30°C is industrially undesirable, because a higher degree of vacuum is required. If the pH is less than 4 during said concentrating step, the aminopolycarboxylate tends to decomposition and precipitation may occur. If the pH exceeds 11, the aqueous solution will be stained and a progressive decomposition of the aminopolycarboxylate may occur.

The thus prepared and obtained aqueous aminopolycarboxylate solution composition enables to stabilize the aminopolycarboxylate, and to prevent troubles such as separation of crystals and solidification of the aqueous solution during the extended time period; the solution maintains a fully stable and homogenous state. This solution is ready and suited for transportation by means of a tank lorry or the like, or for transportation through a pipeline inclusive of piping, valves, nozzles and the like and/or for storing in a tank or the like.

A stabliziation of the solution is especially assured when the aqueous aminopolycarboxylate solution composition is handled under rugged temperature conditions, namely at low temperatures of from -10 to 10°C or at high temperatures of from 30 to 70°C. A handling temperature lower than -10°C drastically decreases the fluidity of the aqueous solution, thus impairing transfer and transportability thereof; a temperature higher than 70°C causes a decomposition of the aminopolycarboxylate thus decreasing the product purity.

According to the most preferred method according to the present invention, the aqueous aminopolycarboxylate solution composition having a molar ratio of the D-form : L-form, of an aspartic acid skeleton of the aminopolycarboxylate of from 1 : 0 to 0.7 : 0.3 or in the range of from 0 : 1 to 0.3 : 0.7 is obtained by reacting aspartic acid and/or a salt thereof with an acrylic acid compound in an aqueous medium under the above specified conditions and is concentrated by heating at a temperature of from 30 to 90°C and at a solution pH of from 4 to 11 to provide an aqueous solution composition having a solid concentration of 25 to 60 mass % and a pH value of not more than 11, and this composition is handled at temperatures of from 30 to 70°C. This mode prevents the staining in the course of concentration, and the aminopolycarboxylate can be handled as a stable and homogeneous aqueous solution free from separation of crystals and in absence of solidification.

This homogeneous aqueous aminopolycarboxylate solution can be stored in a container without providing any special incubation, agitation and other devices. Moreover, it can be delivered directly from the reactor or storage tank to the destination site via a pipeline, or filled in a tank lorry, tank car, container, drum can, or any other transportation means.

During said storage and/or transportation steps, the aqueous aminopolycarboxylate solution is preferably maintained under an inert gas phase, such as nitrogen or argon gas, or air.

The storage tank, for handling the aqueous aminopolycarboxylate solution composition may be made from carbon steel, stainless steel, hastelloy steel, titanium-alloyed steel, nickel steel or the like, and the surface area contacted by the aminopolycarboxylate aqueous solution may be lined with glass, a resin such as Teflon, or rubber. Particularly suited is stainless steel.

The viscosity of the aqueous aminopolycarboxylate solution composition may be adjusted to a value not higher than 10 Pa·s. Such an aqueous aminopolycarboxylate solution composition having a viscosity of not more than 10 Pa-s can be easily handled and constitutes a preferred product as obtainable by the method according to the present invention.

## BEST MODE FOR CARRYING OUT THE PRESENT INVENTION

The following examples illustrate the method according to the present invention in further detail without defining the scope of the invention. Unless otherwise indicated, all "parts" mean "parts by weight", and all "percentages (%)" mean "mass %".

The following equipments were used for the analyses and measurements of the respective components.

Aminopolycarboxylate and impurities other than acrylic polymer: High Performance Liquid Chromatography (HPLC), detector; differential refractometer, ultra violet-visible detector.

Acrylic polymer: Gel Permeation Chromatography (GPC), column; SHODEX Asahipak GF-7M HQ (product name, product of Showa Denko K. K.)

pH: pH Meter F-22 (product name, product of HORIBA, Ltd.), glass composite electrode; 6366-10D (product name, product of HORIBA, Ltd.)

Example 1:

4.56 kg of L-aspartic acid were dissolved in a mixture of 2.7 kg of water and 4.75 kg of 48% aqueous solution of NaOH. While this solution was stirred at the reflux temperature, 3.06 kg of 80% aqueous solution of acrylic acid were added dropwise over 2 hours. After completion of dropwise addition, the stirring at the reflux temperature was continued for 6 further hours. Then, 3.2 kg of water and 1.69 kg of 48% aqueous solution of NaOH were added and the mixture

was cooled to a temperature below 40°C.The thus obtained solution contained 35 mass % of trisodium N-(2-carboxyethyl)-L-aspartate and 8.8 mass % of impurity. The pH of this solution at 25°C was 9.7

Example 2:

The procedure of Example 1 was repeated with the deviation that D-aspartic acid was used in lieu of L-aspartic acid: An aqueous solution was obtained containing 35 mass % of trisodium N-(2-carboxyethyl)-D-aspartate and 8.8 mass % of impurity. The pH of this solution at 25°C was 9.7.

Example 3:

4.56 kg of L-aspartic acid were dissolved in a mixture of 2.7 kg of water and 4.75 kg of 48% aqueous solution of NaOH. While this solution was stirred at the reflux temperature, 3.06 kg of 80% aqueous solution of acrylic acid were added dropwise over 2 hours. After completion of dropwise addition, the stirring at the reflux temperature was continued for 6 further hours. Then, 3.2 kg of water were added and the mixture was cooled to a temperature below 40°C. The thus obtained solution contained 38.2 mass % of trisodium N-(2-carboxyethyl)-L-aspartate and 9.5 mass % of impurity. The pH of this solution at 25°C was 5.2.

Example 4:

The procedure of Example 3 was repeated with the deviation that D-aspartic acid was used in lieu of L-aspartic acid. An aqueous solution was obtained containing 38.3 mass % of trisodium N-(2-carboxyethyl)-D-aspartate and 9.6 mass % of impurity. The pH of this solution at 25°C was 5.1.

Non-inventive Example 5

612 g of L-aspartic acid were dissolved in a mixture of 4000 g of water and 766 g of 48% aqueous solution of NaOH. While this solution was stirred at 30°C, 260 g of acrylonitrile were added dropwise over 1 hour. After completion of dropwise of dropwise addition, the stirring at 30°C was continued for 7 further hours. Then, 437 g of 48% aqueous solution of NaOH were added, and the mixture was heated to 108°C to distil 2440 g of aqueous ammonium out of the system. The thus obtained solution contained 33 mass % of trisodium N-(2-carboxyethyl)-L-aspartate and 3.7 mass % of impurity. The pH of this solution at 25°C was 13.2.

Non-inventive Example 6:

Under cooling, 2.7 kg of water were mixed with 4.75 kg of 48% aqueous solution of NaOH. Thereafter, 4.56 kg of L-aspartic acid were dissolved in said solution. The temperature of this solution was kept at 30°C. With the solution being stirred, 3.06 kg of 80% aqueous solution of acrylic acid were added in a block wise manner and after heating to the reflux temperature, the stirring was continued for 8 further hours. Then, 3.2 kg of water and 1.69 kg of 48% aqueous solution of NaOH were added, and the reaction mixture was cooled to a temperature below 40°C. The thus obtained solution contained 35 mass % of tridosium N-(2-carboxyethyl)-L-aspartate and 8.5 mass % of impurity. Of this amount of impurity, a polymer with a molecular weight of 400000 accounted for 2 mass %. The pH of this solution at 25°C was 10.

Non-inventive Example 7:

An aqueous solution was prepared in the same manner as described in Example 6. 1000 g of 98% sulfuric acid were added under stirring to the obtained solution. Thereafter, the mixture was stirred at 80°C for one hour. After cooling to room temperature, the white crystals were obtained by filtration and were washed with water and dried to give 855 g of N-(2-carboxyethyl)-L-aspartic acid.

Non-inventive Example 8:

The procedure of Example 6 was repeated with the deviation that D-aspartic acid was used in lieu of L-aspartic acid. An aqueous solution was obtained containing 33 mass % of trisodium N-(2-carboxyethyl)-D-aspartate and 3.6 mass % of impurity. The pH of this solution at 25°C was 13.1.

Non-inventive Example 9:

An aqueous solution was prepared in the same manner as described in Example 5. 1000 g of 98% sulfuric acid were gradually added under stirring to the obtained solution. Thereafter, the mixture was stirred at 80°C for one hour. After cooling to room temperature, the white crystals were obtained by filtration and were washed with water and dried to give 850 g of N-(2-carboxyethyl)-L-aspartic acid:

The samples obtained according to Examples 1 to 4 and according to non-inventive Examples 7 to 9 where blended in arbitrary ratios, followed by addition of NaOH where necessary, and the resulting solutions were tested for storage stability. The results are shown in Table 1.

Table 1

| Sample No. | D/L (molar ratio) | Product concentration (mass %) | Impurity (mass %) | Polymer (mass %) | Solid (mass %) | pH at 25°C | Temperature (°C) | Precipitate (after 2 months) |
|---|---|---|---|---|---|---|---|---|
| 1 | 0/1 | 35.0 | 8.8 | Not detected | 43.8 | 9.7 | 10 | None |
| 2 | 0/1 | 35.0 | 8. 8 | Not detected | 43.8 | 9.7 | 25 | None |
| 3 | 0/1 | 35.0 | 8. 8 | Not detected | 43.8 | 9. 7 | 40 | None |
| 4 | 0/1 | 35.0 | 8. 8 | Not detected | 43.8 | 9.7 | 60 | None |
| 5 | 1/0 | 35.0 | 8. 8 | Not detected | 43.8 | 9.7 | 10 | None |
| 6 | 0/1 | 38. 2 | 9.5 | Not detected | 47.7 | 5.2 | 25 | None |
| 7 | 0/1 | 38.2 | 9.5 | Not detected | 47.7 | 5.2 | 40 | None |
| 8 | 0/1 | 38.2 | 9.5 | Not detected | 47.7 | 5.2 | 60 | None |
| 9 | 0/1 | 38.2 | 9.5 | Not detected | 47. 7 | 5.2 | 10 | None |
| 10 | 1/0 | 38.3 | 9.6 | Not detected | 47.9 | 5.1 | 10 | None |
| 11 | 2/8 | 35.0 | 8.8 | Not detected | 43.8 | 9.7 | 10 | None |
| 12 | 8/2 | 35.0 | 8.8 | Not detected | 43.8 | 9.7 | 10 | None |
| 13 | 2/8 | 38.2 | 8.5 | Not detected | 47.7 | 6.2 | 40 | None |
| 14 | 8/2 | 38.3 | 9.6 | Not detected | 47.9 | 6.1 | 40 | None |
| 15 | 3/7 | 35.0 | 8.8 | Not detected | 43.8 | 8.7 | 10 | None |
| 16 | 7/3 | 35.0 | 8.8 | Not detected | 43.8 | 9.7 | 10 | None |
| 17 | 3/7 | 38.2 | 9.5 | Not detected | 47.7 | 5.2 | 40 | None |

(continued)

| Sample No. | D/L (molar ratio) | Product concentration (mass %) | Impurity (mass %) | Polymer (mass %) | Solid (mass %) | pH at 25°C | Temperature (°C) | Precipitate (after 2 months) |
|---|---|---|---|---|---|---|---|---|
| 18 | 7/3 | 38.3 | 9.6 | Not detected | 47.9 | 5.1 | 40 | None |
| 19 | 0/1 | 40 | 6.4 | Not detected | 46.4 | 4.5 | 26 | None |
| 20 | 0/1 | 40 | 6.4 | Not detected | 46.4 | 4.5 | 40 | None |
| 21 | 0/1 | 40 | 6.4 | Not detected | 46.4 | 4.5 | 60 | None |
| 22 | 0/1 | 40 | 8.3 | Not detected | 48.3 | 7.0 | 10 | None |
| 23 | 0/1 | 40 | 8.3 | Not detected | 48.3 | 7.0 | 25 | None |
| 24 | 0/1 | 40 | 8.3 | Not detected | 48.3 | 7.0 | 40 | None |
| 25 | 0/1 | 40 | 8.3 | Not detected | 48.3 | 7.0 | 60 | None |
| 26 | 0/1 | 40 | 9.5 | Not detected | 49.5 | 8.5 | 10 | None |
| 27 | 0/1 | 40 | 9.5 | Not detected | 49.5 | 8.5 | 25 | None |
| 28 | 0/1 | 40 | 8.5 | Not detected | 49.5 | 8.5 | 40 | None |
| 29 | 0/1 | 40 | 9.5 | Not detected | 49.5 | 8.5 | 60 | None |
| 30 | 0/1 | 40 | 10 | Not detected | 60 | 10.0 | 60 | None |
| 31 | 0/1 | 35.0 | 8. 3 | 0.5 | 43.8 | 9.7 | 10 | None |
| 32 | 0/1 | 35.0 | 8.3 | 0.5 | 43.8 | 9.7 | 26 | None |
| 33 | 0/1 | 35. 0 | 8. 3 | 0.5 | 43.8 | 9.7 | 40 | None |
| 34 | 0/1 | 35.0 | 8.3 | 0.5 | 43.8 | 9.7 | 60 | None |

The samples obtained according to non-inventive Examples 5 and 6, were tested for storage stability. The results are shown in Table 2. The samples obtained according to Examples 1 to 3 were tested for storage stability in the as obtained form and/or in concentrated form. The results are also shown in Table 2.

Table 2

| Sample No. | D/L (molar ratio) | Product concentration (mass %) | Impurity (mass %) | Polymer (mass %) | Solid (mass %) | pH at 25°C | Temperature (°C) | Precipitate (after 2 months) |
|---|---|---|---|---|---|---|---|---|
| 35 | 0/1 | 33.0 | 3.7 | Not detected | 36.7 | 13.2 | 25 | White precipitate |
| 36 | 0/1 | 36.0 | 6.5 | 2 | 43.5 | 10 | 25 | Viscous precipitate |

(continued)

| Sample No. | D/L (molar ratio) | Product concentration (mass %) | Impurity (mass %) | Polymer (mass %) | Solid (mass %) | pH at 25°C | Temperature (°C) | Precipitate (after 2 months) |
|---|---|---|---|---|---|---|---|---|
| 37 | 2/8 | 34.5 | 6.2 | 1.9 | 43.8 | 10.2 | 25 | Viscous precipitate |
| 38 | 3/7 | 34.6 | 5.9 | 2.1 | 43.7 | 9.9 | 25 | Viscous precipitate |
| 39 | 4/6 | 35.0 | 8.8 | Not detected | 43.8 | 9.7 | 10 | White precipitate |
| 40 | 6/4 | 35.0 | 8.8 | Not detected | 43.8 | 9.7 | 26 | White precipitate |
| 41 | 5/5 | 35.0 | 8.8 | Not detected | 43.8 | 9.7 | 25 | White precipitate |
| 42 | 5/5 | 38. 2 | 9. 5 | Not detected | 47.7 | 5.2 | 40 | White precipitate |
| 43 | 0/1 | 55 | 6.1 | Not detected | 61.1 | 9.7 | 25 | White precipitate |
| 44 | 0/1 | 56 | 6.1 | Not detected | 61.1 | 9.7 | 40 | White precipitate |

Referring to Table 2 above, the Samples No. 39 to 44 are outside of the range of the present invention with respect to the D-form : L-form ratio; the Samples No. 43 to 44 are outside of the range of the present invention with respect to the solid matter concentration; and the Samples No. 35 is outside of the range of the present invention with respect to the pH. All of these samples showed less stability and provided white precipitates after 2 months storing. The samples No. 36 to 38 were obtained according to Example 6, wherein the aminopolycarboxylate was synthesized by adding the acrylic acid in a block wise manner to the aqueous solution. These samples contained 2 mass % of polymer which adversely affected the stability of these samples.

## Claims

**1.** A method for preparing an aqueous aminopolycarboxylate solution composition containing one or more aminopolycarboxylate compound(s) represented by the following general formula (1):

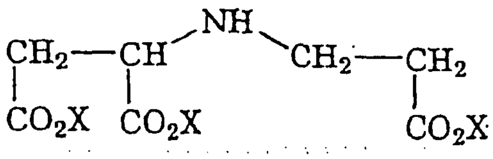

(1)

wherein:

X may be the same or different and represents a hydrogen atom, an alkali metal atom, or an ammonium group;

**wherein said method comprises the steps of:**

- reacting aspartic acid and/or a salt thereof with an acrylic acid compound in an aqueous medium under conditions wherein:

- - the acrylic acid compound is added to the reaction system, and during said adding the temperature of

the reaction system is kept at 50 °C or higher; and

- - the acrylic acid compound is added to the reaction system in such a manner as to adjust the concentration of acrylic acid compound within the reaction system to 10 mass % or less;
- - a content of an acrylic polymer matter formed as impurity from the acrylic acid compound is maintained not higher than 1 mass % based on the whole aqueous solution;
- - the pH of reaction is adjusted to a value not higher than 11 during the reaction or after completing the reaction; and

- providing in the thus prepared aqueous aminopolycarboxylate solution composition a molar ratio of the D-form : L-form of an aspartic acid skeleton of the aminopolycarboxylate in the range of from 1 : 0 to 0.7 : 0.3 or in the range of from 0.3 : 0.7 to 0 : 1 and

wherein the aqueous aminocarboxylate solution composition comprises a solid concentration of from 25 to 60 mass %.

**2.** The method according to claim 1,
**wherein**
the acrylic acid compound is selected from a group including acrylic acid, acrylic ester and sodium acrylate.

**3.** The method according to claim 1,
**wherein**
the acrylic acid compound is added in a dropwise manner to the reaction system.

**4.** The method according to claim 1,
**wherein** the reaction system contains the aspartic acid in a form having 60 to 100% of the carboxylic groups of the aspartic acid neutralized.

**5.** The method according to claim 1,
**wherein**
the acrylic acid compound is added in an amount of 0.8 to 1.2 mol acrylic acid compound per 1 mol aspartic acid and/or salt thereof.

**6.** The method according to claim 1,
**wherein**
the aqueous medium is selected from a group including water, a mixture of water and a solvent soluble in water, and a mixed solvent of water with methanol, ethanol, isopropyl alcohol, acetone or acetonitrile.

**7.** The method according to claim 1,
**wherein**
an aqueous aminocarboxylate solution composition comprising an aminocarboxylate concentration less than 30 mass % is concentrated by heating the aqueous solution under reduced pressure to a temperature of from 30 to 90 °C; and maintaining the pH of the aqueous solution in a range of from 4 to 11.

## Patentansprüche

**1.** Verfahren zur Herstellung einer wässrigen Aminopolycarboxylat-Lösungszusammensetzung,
die eine oder mehrere Aminopolycarboxylat-Verbindung(en) enthält, die der nachstehenden allgemeinen Formel (1) entsprechen:

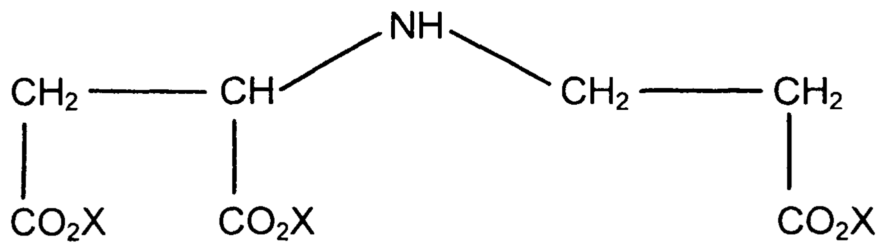

(1)

wobei:

die Gruppen "X" können gleich oder verschieden sein und stehen für ein Wasserstoffatom, für ein Alkalimetallatom oder für eine Ammoniumgruppe; **wobei dieses Verfahren nachstehende Verfahrensschritte aufweist:**

- Asparaginsäure und/oder ein Salz der Asparaginsäure wird mit einer Acrylsäure-Verbindung in einem wässrigen Medium unter nachstehenden Bedingungen umgesetzt:

  - - die Acrylsäure-Verbindung wird zu dem Reaktionssystem hinzugefügt, und während dieser Zugabe wird die Temperatur des Reaktionssystem bei 50 °C oder höher gehalten; und
  - - die Acrylsäure-Verbindung wird zu dem Reaktionssystem in einer solchen Weise hinzugefügt, dass die Konzentration an Acrylsäure-Verbindung im Reaktionssystem 10 Massen-% oder weniger ausmacht;
  - - der Gehalt an Acrylpolymer-Material, das als Verunreinigung aus der Acrylsäure-Verbindung gebildet wird, wird bei einem Wert nicht höher als 1 Massen-% gehalten, bezogen auf die gesamte wässrige Lösung;
  - - während der Umsetzung oder nach Beendigung der Umsetzung wird der pH-Wert des Reaktionsgemisches auf einen Wert nicht höher als 11 eingestellt; und

- in der so hergestellten wässrigen Aminopolycarboxylat-Lösungszusammensetzung wird ein Molverhältnis der D-Form zur L-Form des Asparaginsäure-Gerüstes des Aminopolycarboxylats im Bereich von 1 : 0 bis 0,7 : 0,3 oder im Bereich von 0,3 : 0,7 bis 0 : 1 eingestellt; und wobei die wässrige Aminopolycarboxylat-Lösungszusammensetzung eine Feststoffkonzentration von 25 bis 60 Massen-% aufweist.

**2.** Verfahren nach Anspruch 1,
**dadurch gekennzeichnet, dass**
die Acrylsäure-Verbindung aus einer Gruppe ausgewählt wird, die Acrylsäure einen Acrylsäureester und Natriumacrylat umfasst.

**3.** Verfahren nach Anspruch 1,
**dadurch gekennzeichnet, dass**
die Acrylsäure-Verbindung tropfenweise zu dem Reaktionssystem hinzugefügt wird.

**4.** Verfahren nach Anspruch 1,
**dadurch gekennzeichnet, dass**
das Reaktionssystem die Asparaginsäure in einer solchen Form enthält, bei welcher 60 bis 100 % der Carbonsäuregruppen der Asparaginsäure neutralisiert sind.

**5.** Verfahren nach Anspruch 1,
**dadurch gekennzeichnet, dass**
die Acrylsäure-Verbindung in einem Anteil von 0,8 bis 1,2 Mol Acrylsäure-Verbindung pro Mol Asparaginsäure und/oder Salz der Asparaginsäure hinzugefügt wird.

**6.** Verfahren nach Anspruch 1,
**dadurch gekennzeichnet, dass**
das wässrige Medium aus einer Gruppe ausgewählt wird, die umfasst:

- Wasser;
- eine Mischung aus Wasser mit einem in Wasser löslichen Lösemittel; und
- ein Lösemittelgemisch aus Wasser mit Methanol, Ethanol, Isopropylalkohol, Aceton oder Acetonitril.

**7.** Verfahren nach Anspruch 1,
**dadurch gekennzeichnet, dass**
eine solche wässrige Aminopolycarboxylat-Lösungszusammensetzung, die eine Aminopolycarboxylat-Konzentration kleiner 30 Massen-% aufweist, konzentriert wird, indem diese wässrige Lösung unter vermindertem Druck auf eine Temperatur von 30 bis 90 °C erwärmt wird, wobei der pH-Wert der wässrigen Lösung im Bereich von 4 bis 11 gehalten wird.

**Revendications**

**1.** Un procédé
pour la préparation d'une composition d'une solution aqueuse d'aminopolycarboxylate
qui comprend une ou plusieurs composé(s) d'aminopolycarboxylate répondant à la formule générale suivante (1) :

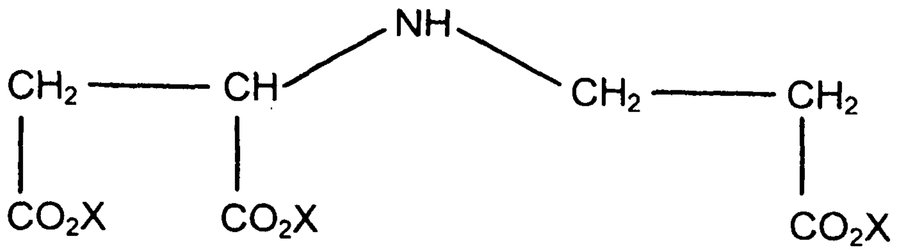

(1)

dans laquelle
le groupe X peut être identique ou différent et représente un atome d'hydrogène, un atome de métal alcalin ou un groupe ammonium ;
**le procédé comprenant les étapes de procédé suivantes :**

- l'acide aspartique et/ou le sel de l'acide aspartique est mis à réagir avec un composé d'acide acrylique dans un milieu aqueux,
et ceci dans les conditions suivantes :

  - - le composé d'acide acrylique est ajouté au système de réaction, et pendant cette addition, la température est maintenue à 50°C ou plus élevée, et
  - - le composé d'acide acrylique est ajouté au système de réaction de manière à ce que la concentration du composé d'acide acrylique dans le système de réaction soit de 10% ou moins en masse, et
  - - la teneur en matière de polymère acrylique qui s'est formée en tant qu'impureté du composé d'acide acrylique est maintenue à une valeur maximale de 1 % en masse par rapport à la solution aqueuse tout entière,
  - - la valeur pH du mélange de réaction étant adaptée pendant la réaction ou à la fin de la réaction à une valeur maximale de 11, et

- dans la composition d'une solution aqueuse d'aminopolycarboxylate ainsi préparée, le rapport molaire forme D / forme L d'un squelette d'acide aspartique de l'aminopolycarboxylate est établi dans la plage de 1 : 0 à 0,7 : 0,3 ou dans la plage de 0,3 : 0,7 à 0 : 1 ;

la composition de la solution aqueuse d'aminopolycarboxylate présentant une concentration de solide de 25 à 60% en masse.

**2.** Le procédé conformément à la revendication 1,

**caractérisé en ce que :**
le composé d'acide acrylique est sélectionné d'un groupe comprenant l'acide acrylique, l'ester acrylique et l'acrylate de sodium.

**3.** Le procédé conformément à la revendication 1,
**caractérisé en ce que :**
le composé d'acide acrylique est ajouté goutte par goutte au système de réaction.

**4.** Le procédé conformément à la revendication 1,
**caractérisé en ce que :**
le système de réaction comprend l'acide aspartique d'une manière dans laquelle 60 à 100% des groupes carboxyliques de l'acide aspartique ont été neutralisés.

**5.** Le procédé conformément à la revendication 1,
**caractérisé en ce que :**
le composé d'acide acrylique est ajouté en une quantité représentant 0,8 à 1,2 moles du composé d'acide acrylique par mole d'acide aspartique et/ou de sel d'acide aspartique.

**6.** Le procédé conformément à la revendication 1,
**caractérisé en ce que :**
le milieu aqueux est choisi d'un groupe comprenant

- l'eau,
- un mélange d'eau et d'un solvant soluble dans l'eau,
et
- un mélange d'un solvant d'eau avec du méthanol, de l'éthanol, d'alcool d'isopropyle, d'acétone ou d'acétonitrile.

**7.** Le procédé conformément à la revendication 1,
**caractérisé en ce que :**
cette composition d'une solution aqueuse d'aminopolycarboxylate dont la concentration en aminopolycarboxylate est inférieure à 30% en masse, est concentrée en échauffant cette solution aqueuse à pression réduite à une température entre 30 et 90°, la valeur pH de la solution aqueuse étant maintenue dans la plage de 4 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI08169866 B
- JP 2644977 B
- EP 0708078 A1
- JP HEI1087580 B
- JP HEI1087582 B
- JP 7224014 A
- JP 10231469 A
- JP 9110812 A
- JP 2002088034 A